# EUROPÄISCHE PATENTSCHRIFT

(11) **EP 0 901 794 B1**
(45) Veröffentlichungstag und Bekanntmachung des Hinweises auf die Patenterteilung: **09.04.2003**
(21) Anmeldenummer: 98116092.2
(22) Anmeldetag: 26.08.1998
(51) Int. Cl.: A61L 9/01

(54) **Duftstoff-enthaltende Zubereitung**
Fragrance composition
Composition parfumée

(30) Priorität: 08.09.1997 DE 19739204
(43) Veröffentlichungstag der Anmeldung: 17.03.1999
(73) Patentinhaber: HAARMANN & REIMER GMBH, D-37601 Holzminden (DE)
(72) Erfinder: Wrede, Wolfgang, 37603 Holzminden (DE); Dröge, Jörg, 37619 Rühle (DE)
(74) Vertreter: Mann, Volker, Dr.

(56) Entgegenhaltungen:
- DE-A- 2 335 111
- DE-A- 3 729 381
- FR-A- 2 680 684
- US-A- 4 322 400

## Beschreibung

Die Erfindung betrifft eine Duftstoff-enthaltende Zubereitung, die beim Gebrauch Duftstoffe an die Umgebung abgibt, so daß sie als Luftverbesserungsmittel einsetzbar ist. Luftverbesserungsmittel (nachfolgend: "LVM") im Sinne der Erfindung sind Produkte, die bereits bei Raumtemperatur (ohne zusätzliche Energiezufuhr) Duftstoffe verdampfen und dadurch die Umgebungsluft parfumieren.

Flüssige LVM sind bekannt (z.B. EP-A 194 017 und 536 444), erfordern aber wegen der Gefahr des Auslaufens eine aufwendige Verpackung und während des Gebrauchs eine sorgfältige Handhabung. Man hat deshalb in der Vergangenheit bereits versucht, diese Nachteile durch Verwendung von LVM in Form weicher Gele oder höherviskoser Pasten zu überwinden (z.B. US-PS 5 324 490). Solche Produkte werden üblicherweise durch Vermischen einer Parfümölemulsion und einer viskositätserhöhenden Komponente hergestellt. Gel- oder pastenartige LVM sollen allerdings viele Forderungen erfüllen.

US-A 4 322 400 offenbart eine feste wasserenthaltende Duftstoffzusammensetzung, die Natriumstearat, Natriumchlorid und Polyglykol enthält und als Luftverbesserungsmittel benutzt werden kann.

Die viskositätserhöhende Komponente soll in möglichst kleiner Menge wirksam sein, bei Raumtemperatur in die Parfumölemulsion einrührbar sein, die Verdampfung der Parfumölkomponenten möglichst wenig verlangsamen und - vor allem - eine möglichst lineare Verdampfung der einzelnen Parfumölkomponenten erlauben, d.h. die Zusammensetzung der verdampfenden Duftstoffe soll möglichst konstant sein.

Die LVM selbst sollen bis zu Temperaturen von 60°C nicht schmelzen; sie sollen auch nicht zu unkontrollierter Migration der wäßrigen Phase und schon gar nicht zu Synärese neigen (auch nicht beim Abkühlen); es soll sich also keine deutlich sichtbare Flüssigkeit auf dem LVM abscheiden. Eine Volumenschrumpfung während des Gebrauchs wird akzeptiert oder sogar gewünscht, weil sie die Verminderung der LVM-Lebensdauer anzeigt.

Bislang sind keine LVM bekannt, die sämtliche genannten Forderungen erfüllen. Aufgabe der Erfindung war es daher, solche verbesserten LVM bereitzustellen.

Überraschenderweise wurde nun gefunden, daß Zubereitungen die obengenannten Bedingungen dann erfüllen, wenn man als Verdickungsmittel sowohl Polymere als auch Aluminiumstearat verwendet. Unter "Aluminiumstearat" werden im Sinne der Erfindung Aluminiummono-, -di- und -tristearate verstanden.

Die neuen Zubereitungen können ohne klassische Emulgatoren verwendet werden. Insofern umfaßt der Begriff "Polymer" anionische Emulgatoren, kationische Emulgatoren und nichtionische Emulgatoren (wie z.B. Polyether) nicht.

Gegenstand der Erfindung sind also wäßrige Zubereitungen enthaltend
- A.: 0,01 bis 5 Gewichtsteile selbstemulgierendes Polymer, wobei das Polymer ausgewählt ist aus der Gruppe der Vinylpolymerisate, Acryl- und Methacrylpolymerisate, Polyamide, Polyimine, Polycarbonsäuren und Polyacrylamide,
- B.: 0,1 bis 1 Gewichtsteile Aluminiumstearat,
- C.: 1 bis 20 Gewichtsteile Parfum und
- D.: 84 bis 97,9 Gewichtsteile Wasser.

Das Polymer A kann in Wasser gelöst und die erhaltene Lösung in geeigneten Kohlenwasserstoffen dispergiert werden. C₁₂-C₂₄-1-Isoolefine haben sich als besonders geeignet erwiesen.

In einer bevorzugten Ausführungsform der Erfindung handelt es sich bei dem Polymer A um ein Polyacrylamid.

Die Zubereitungen können hergestellt werden, indem zunächst das Parfum in das vorgelegte Wasser eingerührt wird. Wenn die Tröpfchen genügend klein geworden sind, was nach einer kurzen Rührzeit mit normalen Rührern der Fall ist, werden das Polymer und das Aluminiumstearat langsam zugegeben, bis die gewünschte Konsistenz erreicht ist. Die Konsistenz ist stabil cremig und kaum noch fließfähig. Die Zubereitungen zeichnen sich durch eine außergewöhnlich lineare Verdampfung des Parfums über einen Zeitraum von mindestens 4 Wochen aus.

Die erfindungsgemäßen Zubereitungen können Färbe- und Konservierungsmittel in üblichen Mengen enthalten.

## Patentansprüche

1. Wässrige Zubereitungen enthaltend
A. 0,01 bis 5 Gewichtsteile selbstemulgierendes Polymer, wobei das Polymer ausgewählt ist aus der Gruppe der Vinylpolymerisate, Acryl- und Methacrylpolymerisate, Polyamide, Polyimine, Polycarbonsäuren und Polyacrylamide,
B. 0,1 bis 1 Gewichtsteile Aluminiumstearat,
C. 1 bis 20 Gewichtsteile Parfum und
D. 84 bis 97,9 Gewichtsteile Wasser.

2. Zubereitungen nach Anspruch 1, worin das Polymer A ein Polyacrylamid ist.

3. Verwendung der Zubereitungen nach den Ansprüchen 1 und 2 als Luftverbesserungsmittel.

## Claims

1. Aqueous preparations containing
A. from 0.01 to 5 parts by weight of self-emulsifying polymer, the polymer being selected from the group of the vinyl polymers, acrylic and methacrylic polymers, polyamides, polyimines, polycarboxylic acids and polyacrylamides,
B. from 0.1 to 1 part by weight of aluminium stearate,
C. from 1 to 20 parts by weight of perfume, and
D. from 84 to 97.9 parts by weight of water.

2. Preparations according to claim 1, wherein the polymer A is a polyacrylamide.

3. Use of the preparations according to claims 1 and 2 as air fresheners.

## Revendications

1. Préparations aqueuses contenant
A. 0,01 à 5 parties en poids de polymère auto-émulsifiant, le polymère étant choisi dans le groupe des polymères vinyliques, des polymères acryliques et méthacryliques, des polyamides, polyimines, acides polycarboxyliques et polyacrylamides,
B. 0,1 à 1 partie en poids de stéarate d'aluminium,
C. 1 à 20 parties en poids de parfum et
D. 84 à 97,9 parties en poids d'eau.

2. Préparations selon la revendication 1, dans lesquelles le polymère A est un polyacrylamide.

3. Utilisation des préparations selon les revendications 1 et 2 comme agent améliorant l'air.
